# EUROPÄISCHE PATENTSCHRIFT

(11) **EP 1 404 264 B1**
(45) Veröffentlichungstag und Bekanntmachung des Hinweises auf die Patenterteilung: **23.01.2019**
(21) Anmeldenummer: 02764682.7
(22) Anmeldetag: 12.07.2002
(51) Int. Cl.: A61F 9/00

(54) **INSERT ZUR BEHANDLUNG DES TROCKENEN AUGES**
INSERT FOR THE TREATMENT OF DRY EYE
INSERT UTILISÉ DANS LE TRAITEMENT DE LA SÉCHERESSE OCULAIRE

(30) Priorität: 12.07.2001 DE 10133870
(43) Veröffentlichungstag der Anmeldung: 07.04.2004
(73) Patentinhaber: Pharma Stulln GmbH, 92551 Stulln (DE)
(72) Erfinder: LOHMANN, Chris P., 93138 Lappersdorf (DE); GÖPFERICH, Achim, Lehrstuhl Pharmazeut. Technol., 93040 Regensburg (DE); KOELWEL, Christoph, 93059 Regensburg (DE)
(74) Vertreter: DREISS Patentanwälte PartG mbB
(86) Internationale Anmeldenummer: PCT/EP2002/007805
(87) Internationale Veröffentlichungsnummer: WO 2003/005941

(56) Entgegenhaltungen:
- EP-A- 0 364 266
- EP-A2- 0 251 680
- WO-A1-94/05257
- US-A- 3 986 510
- US-A- 4 923 700
- US-A- 5 137 728
- US-A- 5 378 475
- US-A- 5 518 732
- US-A- 5 660 851
- US-A- 5 989 579

## Beschreibung

Die Erfindung betrifft die Verwendung eines Inserts zur Verabreichung von EGF am Auge zur Behandlung des sogenannten "Dry Eye Syndrome".

Im Vergleich zu gesunden Personen weisen Patienten, die an dem Dry Eye Syndrom leiden, eine geringere Produktion von Epidermal Growth Factor (EGF) in den Epithelzellen der Hornhaut verglichen mit dem physiologischen Niveau auf.

Vor ca. 50 Jahren beschrieb Henrik Sjögren eine Krankheit hervorgerufen durch einen Autoimmunschaden des Tränendrüsengewebes, verringerter Tränensekretion und Augenoberflächenerkrankung und er nannte diese Krankheit Keratokonjunktivitis Sicca (KCS). Es wurde zwischenzeitlich herausgefunden, dass KCS oder "Dry Eye" Bestandteil einer Anzahl von Fehlfunktionen ist, die aus jedem Umstand herrühren, der die Tränensekretion senkt oder die Verdunstung des Tränenfilms steigert.

In der Zeitschrift Cornea 12, 202, 1994 haben Tsubota et al. Patienten mit Dry Eye Disease in drei Gruppen einklassifizert, nämlich (1) trockene Augen in Verbindung mit dem Sjögren-Syndrom, (2) autoimmun positive trockene Augen (zirkulierende Antikörper) und (3) einfache trockene Augen (jede Art von Augenoberflächenfehlfunktionen; nicht in Verbindung mit einer Autoimmunkrankheit).

Die Dry Eye-Krankheit ruft typischerweise Augenirritationen hervor, ebenso wie sie durch Fremdkörper im Auge verursacht werden, wie Augenschmerzen, Augenbrennen und ähnliches.

Die klinischen Symptome von KCS sind ein Resultat der pathologisch veränderten Epitheloberfläche der Hornhaut von KCS-Patienten. Es ist bekannt, dass das Hornhautepithel von KCS-Patienten eine Anzahl von Unterschieden zeigt im Vergleich zu dem Epithelium von gesunden Personen, nämlich (1) der normale Epithelumsatz ist verändert; (2) die Dicke der Epithelschicht ist nicht gleichmäßig; (3) die Oberfläche des Epithel ist unregelmäßig; (4) interzellulare Verbindungen, wie Hemidesmosome, sind nicht ausreichend vorhanden in Quantität und Qualität.

Heutzutage wird die Dry Eye-Krankheit üblicherweise mit sogenannten öligen Augentropfen oder Einlagen behandelt, die sich langsam auflösen, auch bekannt als künstliche Tränen. In den meisten Fällen ist der Behandlungserfolg nicht zufriedenstellend. Dieser Misserfolg ist u.a. durch die kurze Verweilzeit derartiger Augentropfen zu erklären. Erste derartige Augenpräparate zur Behandlung des Dry Eye-Syndroms wurden in den 70er-Jahren entwickelt, als wässrige Lösungen umfassend Polymere, wie Polyvinylalkohol, Zellulosederivate oder Polyvinylpyrrolidone oder andere Polymere dank ihres öligen Effekts (US-PS 4,120,949, US-PS 4,744,980; US-PS 4,883,658; US-PS 5,209,927; und US-PS 5,770,628).

Es wurden weiterhin "künstliche Tränen" entwickelt, die Matrizes umfassten, bestehend aus wasserlöslichen Polymeren, die sich nach der Platzierung im Tränensack langsam auflösten (US-PS 4,343,738).

Abgesehen von Lösungen und festen Inserts wurden auch halbfeste Präparate entwickelt, die Polymere umfassten, um einen gewissen öligen Effekt zu erzeugen (US-PS 5,075,104).

Ein anderer Ansatz zur Behandlung des Dry Eye-Syndroms war die Entwicklung von Substanzen, die die Produktion von Tränenflüssigkeit stimulieren (US-PS 4,820,737). Darüber hinaus wurden auch andere Arzneistoffe, wie Retinoide (US-PS 4,826,871; US-PS 4,966,773; und US-PS 5,185,372) sowie Kalziumsalze (US-PS 5,290,572; US-PS 5,595,764) und Steroide (US-PS 5,041,434) für die Behandlung der Krankheit getestet. Keiner der Ansätze führte zu einer signifikanten Verbesserung der Hornhautveränderungen, wie sie zuvor beschrieben wurden. Sie erlaubten lediglich, die Symptome über einen kurzen Zeitraum zu behandeln, die auf die Gewebezerstörung folgen.

Ähnliche Probleme mit der kurzen Zeit während derer eine Augentherapie effektiv ist, wurden festgestellt, wenn Arzneistoffe an das Auge abgegeben werden sollen. Augentropfen besitzen lediglich eine kurze Zeitspanne, ca. 30 Sekunden, während dessen sie sich im Auge aufhalten. Um dieses Problem zu überwinden, wurden in den frühen 70ern die ersten Arzneimittel entwickelt, die Arzneistoffe über einen verlängerten Zeitraum abgeben. Diese Inserte wurden in den Tränensack eingelegt und waren ursprünglich aus Materialien, die sich nicht abbauten und/oder zersetzten und/oder während der Verwendung auflösten. Sie mussten daher konsequenterweise nach der Behandlungsperiode aus dem Auge entfernt werden (US-PS 3,618,604; US-PS 4,057,619). Einige Systeme wurden derart ausgestaltet, dass das Entfernen der Materialien erleichtert wurde, beispielsweise durch Vorsehen von magnetisch anziehbaren Materialien (US-PS 3,626,940). Die Abgabe der Arzneistoffe war durch die Verwendung von festen Trägermaterialien auf die Diffusion beschränkt. Hierdurch konnte die Abgaberate durch Verwendung von mikroporösem Material kontrolliert werden (US-PS 3,828,777). Des weiteren wurden biologisch zersetzbare Materialien für Inserte verwendet, die sich während der Benutzung im Auge auflösten (US-PS 3,867,519; US-PS 4,179,497). Diese Inserte hatten den Vorteil, dass eine Entfernung nach Ende der Behandlung nicht notwendig war.

Weitere Entwicklungen umfassten die Verwendung von Lipiden im Gegensatz zu Polymeren als Material für die Verwendung von Inserte (US-PS 3,968,201), die Verbesserung und Anpassung der Geometrie und der mechanischen Eigenschaften zur Verbesserung der Anwendung (US-PS 3,963,025) sowie spezielle Einsätze mit Befestigungseinheiten (US-PS 5,395,618).

Schließlich ist aus der EP 0 251 680 ein Insert umfassend einen freisetzbaren Wirkstoff, nämlich Epidermal Growth Factor (EGF) bekannt, das hier zur Verbesserung der Wundheilung dienen soll. Das Insert umfasst hierbei eine sich schnell auflösende Matrix sowie in der Matrix vorgesehene Mikropartikel, die entweder als Hydrogele enthaltend den Wirkstoff aufgebaut sein können, oder auch als Matrixmaterialien oder als Materialien umfassend eine Membran, in der beispielsweise eine Flüssigkeit mit dem Wirkstoff enthalten ist.

Ausgehend hiervon ist es Aufgabe der Erfindung, die Behandlung von Dry Eye-Patienten zu verbessern.

Die Aufgabe wird durch die Patentansprüche 1 bis 3 gelöst.

Es wurde dabei festgestellt, dass EGF ein wirksames Therapeutikum zur Behandlung von Augen darstellt. Es wurden erfindungsgemäß Pharmazeutika entwickelt, die das EGF-Niveau im Auge anheben und sogar noch entscheidender EGF und Kalzium mit einer bevorzugt linearen Kinetik über einen Zeitraum von mehreren Stunden freisetzen. Dies ist notwendig, um die Epithelzellen der Hornhaut zu stimulieren um Hemidesmosome und andere interzellulare Adhäsionsmoleküle zu synthetisieren und diese Zell-Zell-Verbindugen zu ermöglichen.

Es wurde überraschenderweise festgestellt, dass diese Präparate in der Lage sind, die Therapie von Patienten mit trockenen Augen deutlich zu verbessern. Darüber hinaus wird ein Verabreichungssystem für Medikamente, die eine bessere Zuführung von EGF an das Auge erlauben beschrieben.

Die Architektur, der normale physiologische Umsatz und, wenn notwendig, der Wundheilungsprozess des Hornhautepithels werden durch Zytokine kontrolliert. Unter einer Vielzahl von Zytokinen erscheint EGF recht wichtig für das Hornhautepithelium in Verbindung mit den zuvor genannten physiologischen Funktionen. EGF ist verantwortlich für die Erhaltung des Hornhautepitheliums und wird in der Tränendrüse und in den Basalzellen des Hornhautepithels hergestellt. Im Auge befindet sich der EGF-Rezeptor im Epithel der Hornhaut, der Linse sowie dem Endothel der Hornhaut. Die Rezeptoren haben eine hohe Affinität zu EGF und sind sättigbar. In verletzten Augen steigt die Dichte der Epithelrezeptoren von EGF. EGF wurde daher vorgeschlagen zur Behandlung von verletzten Augen. Trotz des positiven Effekts von EGF auf das Hornhautepithel ist EGF, als Augentropfen verabreicht, nicht als Arzneistoff zur Behandlung des Dry Eye-Syndroms angesehen worden.

Alleinig Serumpräparate, die eine Vielzahl von anderen Zytokinen außer EGF beinhalten, sind seither in der Dry Eye Syndrom-Therapie getestet worden.

Bei Dry Eye-Patienten liegt das Niveau von EGF in der Tränenflüssigkeit und in den Hornhautepithelzellen deutlich niedriger als bei normalen Patienten. Hierzu wurden Tränenflüssigkeitsproben genommen. Die Probeentnahme erfolgte unter Ausnutzung des Kapillareffekts. Die Kapillarröhrchen wurden in einem Winkel von 10 bis 30° oberhalb der horizontalen Achse und in einem Winkel von 10 bis 40° zu der Oberfläche der unteren Fornix gehalten. Die Spitze des Röhrchens berührte den Tränenflüssigkeitsspiegel und wurde langsam auf dem Fornix Inferior von der Mittellinie in Richtung des lateralen Augenwinkels geführt. Es wurde dabei sorgfältig darauf geachtet, nicht die Oberfläche des Augapfels beim Zwinkern zu berühren und das Röhrchen wurde schnell aus dem Fornix entfernt. Alle Proben wurden unmittelbar in Eppendorf-Röhrchen überführt, mit Trockeneis gefroren und bei -70° C bis zur Bestimmung der EGF-Konzentration gelagert. Um verschiedene individuelle Proben zu vergleichen, wurde die Zeit für die Probenahme gemessen und mit dem EGF-Gehalt korreliert. Die EGF-Konzentration in der Tränenflüssigkeit wurde unter Verwendung eines ultrasensitiven immunofluorometrischen ELISA-Tests vom Sandwich-Typ (R&D Systems, Minneapolis, USA) bestimmt. In dieser zweistufigen Feststoffphasentechnik wird immunoreaktives EGF zuerst an einen polyklonalen anti-EGF Antikörper gebunden, der an die feste Phase geheftet ist und dann unter Verwendung eines monoklonalen Anti-Human EGF Antikörpers quantifiziert. Die kleinste bestimmbare Menge von EGF bei diesem Test beträgt 0,2 pg/ml für unverdünnte Proben und 40 pg/ml für verdünnte Proben.

Die folgend Tabelle 1 zeigt die Resultate der analysierten EGF-Mengen in der Tränenflüssigkeit von normalen Patienten und Dry Eye-Patienten.

**TABELLE 1**

| | |
|---|---|
| Gesunde (n=30) | 1,7 - 4,3 ng/ml |
| Patienten mit primärer KCS (n=30) | 0,02 - 0,03 ng/ml |
| Patienten mit Sjörgren's Syndrom (n=30) | 0,02 - 0,22 ng/ml |
| Patienten mit Meibomitis (n=30) | 0,3 - 0,95 ng/ml. |

In einem weiteren Experiment wurden Hornhautepithelzellen für molekularbiologische Untersuchungen hinsichtlich der individuellen Herstellung oder Synthese von EGF betrachtet durch Messung der EGF mRNA. Ein kleines Gewebestück von 0,8 mm Durchmesser wurde in einer Entfernung von ca. 2 mm vom Limbus entnommen. Das Stück wurde unmittelbar nach der Entnahme in ein Eppendorf-Röhrchen transferiert zusammen mit 300 µl von RNAzol (WAK Chemie, Heidelberg, Deutschland). Die quantitative Analyse des EGF wurde nach einer bekannten Technik durchgeführt, bei der mRNA für Epidermal Growth Factor mit PCR (Polymerase Chain Reaction) verstärkt und dann analysiert wird. Tabelle 2 zeigt die Resultate der EGF mRNA-Niveaus, die in den Hornhautepithelzellen von normalen Personen und Dry Eye-Patienten analysiert wurden.

**TABELLE 2**

| | |
|---|---|
| Gesunde (n=30) | 6,25 - 13,11 Einheiten |
| Patienten mit primärer KCS (n=30) | 0,23 - 1,34 Einheiten |
| Patienten mit Sjögren's Syndrom (n=30) | 0,31 - 1,11 Einheiten |
| Patienten mit Meibomitis (n=30) | 2,78 - 6,69 Einheiten. |

Die vorstehenden Resultate zeigen, dass Patienten, die unter primärem Dry Eye-Syndrom (primäres KCS) leiden und Patienten mit Sjögren's-Syndrom deutlich verringerte EGF-Niveaus besitzen. Es wird vermutet, dass diese gesenkten lokalen EGF-Niveaus für die Veränderungen im Bereich der Hornhaut verantwortlich sind und letztendlich zum Dry Eye-Syndrom führen.

Basierend auf diesen Resultaten wurden eine histologische und zwei klinische Studien durchgeführt. Bei der histologischen Studie wurden menschliche Hornhäute durchgehend einer Lösung, die 5 % EGF enthielt, über verschiedene Zeiträume, zwischen 30 Sekunden und 4 Stunden, ausgesetzt. Nach einer Kontaktzeit von 30 Sekunden, die in etwa der Zeit entspricht, in der Augentropfen in Kontakt mit der Hornhautoberfläche stehen, wurde eine schwache Epithelzellarchitektur mit nur wenigen interzellularen Adhäsionsmolekülen gefunden. Im Gegensatz dazu wurde nach einer Kontaktzeit von 4 Stunden mit EGF auf der Hornhautepitheloberfläche festgestellt, dass das Epithel eine optimale Architektur aufwies, mit vielen interzellularen Adhäsionsmolekülen, wie beispielsweise Hemidesmosomen. In einer klinischen Studie unter Verwendung von 5 % EGF-Augentropfen wurde kein positiver Effekt auf die Symptome der Patienten oder die morphologischen Anzeichen bei 13 Patienten gefunden. Im Gegensatz dazu wurde bei einer durchgehenden Anwendung von 5 % EGF gelöst in Methylzellulose über 4 Stunden eine deutliche Verbesserung der Patientensymptome sowie der morphologischen Anzeichen des Hornhautepithels für 5 bis 7 Tage festgestellt.

Abgesehen von der Feststellung, dass EGF ein sinnvoller Wirkstoff für die Verwendung zur Behandlung von Dry Eye-Patienten darstellt, durch die Induzierung der Synthese von Zell-zu-Zell-Adhäsionsmolekülen stellt ein weiterer Hauptaspekt der Erfindung die Entwicklung eines Arzneimittelabgabesystems dar, das es erlaubt, EGF-defiziente Patienten mit dieser Art von Wachstumsfaktor zu versorgen.

Obwohl die lokale Verabreichung von EGF im Auge der hauptsächliche Ansatz ist, mag es in der Zukunft sinnvoll erscheinen, EGF systemisch über eine Vielzahl verschiedener Eingabewege zu verabreichen, (beispielsweise oral oder parenteral). Es ist außerdem denkbar, Zellen der Tränendrüse oder Stammzellen des Hornhautepithels so zu verändern, dass sie EGF produzieren oder solche EGF-produzierenden Zellen zu transplantieren.

Dabei soll der Begriff "EGF" im Rahmen dieser Erfindung vorzugsweise menschliches EGF bedeuten, aber es kann auch EGF von anderen Lebewesen oder Substanzen, die an den EGF-Rezeptor binden, oder chemische Modifikationen derartiger Substanzen wie beispielsweise PEGylierte Substanzen bedeuten. Menschliches EGF ist eine 6,045 kD Polypeptid-Kette aus 53 Aminosäuren mit drei Disulfid-Bindungen innerhalb der Kette. EGF wurde seither lediglich vorgeschlagen als therapeutisches Mittel zur Beeinflussung der Wundheilung und insbesondere nach Hornhaut- und refraktiven Operationen.

Daher ist es ein weiterer Gegenstand der vorliegenden Offenbarung, ein Trägersystem für die Verabreichung von EGF an seinem Angriffspunkt bereitzustellen. Von großer Bedeutung ist die durchgehende Verabreichung von EGF an die Hornhautepitheloberfläche.

Ophthalmika, die verwendet werden können, um EGF lokal an das Auge abzugeben, sind Tropfen, Salben, Gele oder feste Inserte. Derartige Präparationen umfassen die aktive Komponente EGF im Bereich von 0,000'0001 bis ca. 20 Gew.-%, vorzugsweise von ca. 0,000'1 bis ca. 1 Gew.-% und besonders bevorzugt von 0,01 bis 0,5 Gew-% oder in solch einer Menge, dass das EGF-Niveau in der Tränenflüssigkeit auf das physiologische Niveau angehoben wird. Die Dosis der aktiven Komponente kann von verschiedenen Faktoren abhängen, wie beispielsweise die Art der Verabreichung, Bedarf, Alter oder persönliches Befinden.

Die Verabreichung mittels Augentropfen ermöglicht zwei verschiedene prinzipielle Wege: Die Auflösung von EGF in Wasser oder die Applikation als Suspension in z. B. öligen Trägern.

Es existieren eine Reihe von Additiven, die verwendet werden können, um Tropfen herzustellen. Beispiele für pharmazeutische akzeptable Trägerstoffe und Additive sind bekannt und nachstehend aufgeführt. Sie umfassen einen Träger, isotonische Agenzien, Pufferlösungen, Komplexbildner, Lösungsmittel und Verdickungsmittel. Beispiele für derartige Träger und Additive können darüber hinaus auch WO 91/15206 oder der pharmazeutischen Literatur entnommen werden.

Für die Herstellung von wässrigen EGF-Lösungen wird die aktive Komponente in einer sterilen, wässrigen isotonischen Lösung gelöst, die, sofern notwendig, auf den gewünschten pH-Wert gepuffert wird.

Ein Opthalmikum umfassend EGF beinhaltet gewöhnlich einen Träger. Ein weiterer Aspekt der vorliegenden Offenbarung ist daher auf ein Ophthalmikum umfassend EGF und einen Träger gerichtet.

Die Offenbarung betrifft des weiteren ein Ophthalmikum umfassend EGF, einen Träger sowie ein Lösungsmittel.

Mögliche Träger sind inbesondere Gelatine, Traganth, Methylzellulose und/oder Polyvinylpyrrolidon, Agar oder Alginsäure oder deren Salze, wie beispielsweise Natriumalginat, Gummi Arabicum, Polyvinylpyrrolidon, Polyethylenglykol. Für die Verabreichung mittels Tropfen sind z. B. wässrige Lösungen von EGF oder z. B. Suspensionen von EGF in Ölen geeignet. Geeignete lipophile Lösungsmittel oder Träger sind fette Öle, wie beispielsweise Sesamöl oder synthetische Fettsäureester, z. B. Ethyloleat oder Triglyzeride oder wässrige Injektionssuspensionen, die die Viskosität erhöhende Substanzen enthalten, wie beispielsweise Natriumcarboxymethylzellulose, Sorbitol und/oder Dextran und, sofern gewünscht, auch Stabilisatoren.

Träger, die in Verbindung mit der vorliegenden Erfindung benutzt werden, sind z. B. Wasser oder Mischungen von Wasser und wassermischbaren Lösungsmitteln, wie beispielsweise C₁- bis C₇-Alkohole, pflanzliche Öle oder Mineralöle, die zwischen 0,5 und 5 Gew.-% Hydroxyethylzellulose, Ethyloleat, CarboxymethylZellulose, Polyvinylpyrrolidon oder nicht toxische, wasserlösliche Polymere für den ophthalmischen Gebrauch enthalten, wie z. B. Zellulosederivate, wie beispielsweise Methylzellulose, Alkalimetallsalze der Carboxymethylzellulose, Hydroxymethylzellulose, Hydroxyethylzellulose, Methylhydroxypropylzellulose, Hydroxypropyzellulose, Chitosan, Skleroglukan, Acrylat oder Methacrylat, wie beispielsweise die Salze der Polyacrylsäure oder Ethacrylate, Polyacrylamide, natürliche Produkte, wie beispielsweise Gelatine, Alginat, Pektin, Traganth, Karaya Gummi, Xanthan Gummi, Karragenan, Agar und Gum Arabicum, Stärkederivate, wie beispielsweise Stärkeazetat, Hydroxylpropylstärke und auch andere synthetische Produkte, wie beispielsweise Poloxamer, z. B. Poloxamer F127, Polyvinylalkohol, Polyvinylpyrrolidon, Polyvinylmethylether, Polyethylenoxid, vorzugsweise vernetzte Polyacrylsäure, wie beispielsweise neutrales Carbopol, oder Mischungen dieser Polymere. Bevorzugter Träger ist Wasser. Zellulosederivate, sind beispielsweise Methylzellulose, Alkalimetallsalze der Carboxymethylzellulose, Hydroxymethylzellulose, Hydroxyethylcellulose, Methylhydroxypropylzellulose und Hydroxypropylzellulose, neutrales Carbopol oder Mischungen hiervon. Die Konzentration der Träger beträgt beispielsweise zwischen dem 0,1 und dem 100.000-Fachen der Konzentration der aktiven Komponente.

Lösungsvermittler und ähnliche Hilfstoffe können ebenfalls in einem Ophthalmikum der vorliegenden Erfindung verwendet werden und sind beispielsweise Tyloxapol, Fettsäureglycerol-Polyethylen-Glycolester, Fettsäurepolyethylen-Glycolester, Fettsäure-Polyethylen-Glycolester, Polyethylenglycol, Polyglycerolester, Polysorbat 20, Polysorbat 80 oder Mischungen aus diesen Komponenten. Ein besonderes Beispiel für ein insbesondere bevorzugtes Lösungsmittel ist ein Reaktionsprodukt aus Rhizinusöl und Ethylenoxid, z. B. das kommerziell erhältliche Produkt Cremophor EL® oder Cremophor RH 40®. Reaktionsprodukte von Rhizinusöl und Ethylenoxid haben sich als besonders gute Lösungsmittel herausgestellt, die ausgesprochen gut vom Auge vertragen werden. Ein anderes bevorzugtes Lösungsmittel ist Tyloxapol. Die Konzentrationen, die verwendet werden, hängen insbesondere von der Konzentration der aktiven Komponente ab. Die zugefügte Menge ist typischerweise ausreichend, um die aktive Komponente zu lösen. Z. B. kann die Konzentration des Lösungsmittels zwischen dem 0,1 bis dem 5.000-Fachen der Konzentration der aktiven Komponente betragen.

Elektrolyte, die für die Formulierung interessant sind, umfassen Substanzen, die es erlauben, den pH-Wert während der Lagerung oder der Verwendung der Formulierung einzustellen, sowie Elekrolyte, die die Proteinstruktur und daher die Stabilität von EGF beeinflussen. Beispiele für Pufferlösungen sind Azetat, Ascorbat, Borat, Hydrogenkarbonat/Karbonat, Zitrat, Glukonat, Laktat, Phosphat, Propionat und TRIS-Puffer. Tromethamine und Boratpuffer sind die bevorzugten Pufferlösungen. Die zugefügte Menge an Pufferlösung kann beispielsweise der Menge entsprechen, die notwendig ist, um einen physiologisch tolerierbaren pH-Bereich einzustellen und zu erhalten. Der pH-Bereich ist typischerweise im Bereich von 5 bis 9, vorzugsweise zwischen 6 bis 8,5 und insbesondere zwischen 6,5 und 8,2 angesiedelt.

Elektrolyte können darüber hinaus den osmotischen Druck und die Proteinkonformation beeinflussen. Derartige Elektrolyte sind z. B. ionische Komponenten, beispielsweise Alkalisalze oder Erdalkalihalogenide, wie beispielsweise CaCl₂, KBr, KCI, LiCI, NaI, NaBr oder NaCI, Na₂SO₄ oder Borsäure. Nichtionische, die Tonizität verstärkende Agentien sind beispielsweise Harnstoff, Glyzerol, Sorbitol, Mannitol, Propylenglykol oder Dextrose. Beispielsweise werden in ausreichender Menge die Tonizität verstärkende Agentien zugesetzt, um einer gebrauchsfertigen ophthalmischen Präparation eine Osmolalität von ca. 50 bis 1000 mOsmol, vorzugsweise von 100 bis 400 mOsmol, und insbesondere zwischen 200 bis 400 mOsmol und insbesondere zwischen 250 bis 350 mOsmol zu verleihen.

Beispiele für Konservierungsstoffe sind quaternäre Ammoniumsalze, wie beispielsweise Sepazoniumchlorid, Zethyltrimethylammonium-Bromid (Zetrimide), Zetylpyridinium-Chlorid, Benzoxonium-Chlorid, Benzethonium-Chlorid, Domiphenbromid (Bradosol®) oder Benzalkonium-Chlorid, Alkylquecksilbersalze der Thiosalicylsäure, wie beispielsweise Thiomersal, Phenylquecksilbernitrat, Phenylquecksilberazetat oder Phenylquecksilberborat, Parabene, wie beispielsweise Methlyparaben oder Propylparaben, Alkohole, wie beispielsweise Chlorobutanol, Benzylalkohol oder Phenylethanol, Guanidinderivate, wie beispielsweise Chlorohexadin, oder Polyhexamethlyen-Biguanid, Sodiumperkabonat, Germal® oder Sorbinsäure. Bevorzugte Konservierungsmittel sind quaternäre Ammoniumsalze, Alkyl-Quecksilbersalze und Parabene. Wo angezeigt, kann eine ausreichende Menge von Konservierungsmitteln zu den Ophthalmika hinzugefügt werden, um einen Schutz gegen Sekundärkontamination während der Verwendung, hervorgerufen durch Bakterien und Pilze, sicherzustellen.

Die Ophthalmika können darüber hinaus weitere nicht toxische Trägerstoffe, wie beispielsweise Befeuchtungsmittel oder Füllstoffe, wie beispielsweise Polyethylenglykole oder Derivate davon (wie beispielsweise MethylPEG oder PEGamine) enthalten mit einem Molekulargewicht von 200 bis 10.000 oder mehr. Andere Trägerstoffe, die verwendet werden können, sofern gewünscht, sind nachfolgend aufgeführt, wobei diese Aufzählung keine Beschränkung der möglichen Trägerstoffe darstellen soll. Es handelt sich hierbei insbesondere um Komplexbildner, wie beispielsweise Disodium-EDTA oder EDTA, Antioxidantien, wie beispielsweise Ascorbinsäure, Azetylcystein, Cystein, Natriumhydrogensulfid, Butyl-Hydroxyanisol, Butylhydroxytoluen oder Alpha-Tocopherol-Acetat, Stabilisatoren, wie beispielsweise Thiourea, Thiosorbitol, Natrium-Dioctyl-Sulfosuccinat oder Monothioglyzerol oder andere Trägerstoffe, wie beispielsweise Laurinsäuresorbitolester, Triethanolaminoleat oder Palmsäureester. Bevorzugte Trägerstoffe sind Komplexbildner wie Disodium-EDTA. Die Menge und die Art der zugefügten Trägerstoffe hängt ab von den besonderen Erfordernissen und liegt generell im Bereich zwischen ungefähr 0,0001 bis ungefähr 90 Gew.-%.

Die Erfindung betrifft Inserte zur Verwendung im Auge.

EGF kann an das Auge abgegeben werden über kleine Vorrichtungen, die in den Tränensack des Auges eingelegt werden. Derartige Systeme geben EGF über eine Periode zwischen einer Stunde und zwei Wochen frei. Ein besonders bevorzugter Zeitraum für die Freigabe beträgt vier Stunden bis eine Woche. Die Form derartiger Inserte ist an die Anatomie und Physiologie des Auges angepasst. Die Geometrie der Inserte kann beispielsweise zylindrisch rund oder oval oder jede andere Form, die geeignet ist, um in den Tränensack des Auges eingelegt zu werden, besitzen. Darüber hinaus kann das Insert auch die Form einer Kontaktlinse haben, wobei sie in diesem Fall auf die Hornhaut des Auges aufgelegt wird. Die bevorzugte Form des Inserts ist rund oder oval mit einem ersten Durchmesser von r₁ zwischen 0,1 mm und 20 mm, und einem zweiten Durchmesser r₂ zwischen 0,1 mm und 20 und einer Dicke d zwischen 5 µm und 5 mm. Besonders bevorzugt ist eine ovale Geometrie mit r₁ = 0,5 mm bis 18 mm, r₂ = 0,5 mm bis 10 mm und einer Dicke d = 10 µm bis 1 mm. Eine darüber hinausgehende wichtige Charakteristik besteht darin, dass alle Inserte Calziumionen zusammen mit dem EGF freigeben können, da herausgefunden wurde, dass Calzium für die Verbesserung des Zell-Zell-Kontakts im Hornhautepithel notwendig ist.

EGF kann von dünnen Filmen freigegeben werden, die beispielsweise aus Polymerfilmen bestehen, in welche EGF eingebettet ist. EGF kann in der Matrix gelöst oder suspendiert sein. Derartige Filme besitzen den Vorteil, dass sie einfach herzustellen sind im großen Maßstab und als individuelle Inserte ausgeschnitten oder aus einem größeren Film in jeder gewünschten Geometrie ausgestanzt werden können.

Einschichtige Filme aus EGF bestehen aus einem einzigen Matrixmaterial bzw. Materialgemisch, in welches EGF eingebettet ist. Eine einfache Methode, derartige Matrizes für die lokale EGF-Freigabe im Auge zu erhalten, besteht darin, das Protein in einer Lösung oder in einem geschmolzenen Matrixmaterial zu dispergieren. Durch Verdunstung des Lösungsmittels oder durch Abkühlung der Schmelze erhält man eine mit EGF angereicherte Matrix. Ein Beispiel für eine derartige Herstellung sind Hydrogele. EGF kann z. B. in einer 1 %-igen (w/w) wässrigen Alginatlösung gelöst werden. Nach dem Gießen in Formen mit einem definierten Oberflächenbereich wird das Hydrogel getrocknet, um einen festen Film zu bilden, aus dem die Inserte durch Schneiden oder Stanzen von Stücken mit gewünschter Geometrie erhalten werden. Die Filmdicke kann durch Steigerung des Alginatgehaltes oder durch Reduktion der Formoberfläche variiert werden.

Abhängig vom gewählten Matrixmaterial können die Inserte nicht errodierbar oder errodierbar sein. Im zuletzt genannten Fall besteht keine Notwendigkeit, das Insert nach der Verwendung zu entfernen. Materialien, die benutzt werden können für die Herstellung von nicht errodierbaren Inserten, sind beispielsweise Polyacrylate oder Ethylenvinylazetat-Copolymere. Solche Matrizes können mit wasserlöslichen Substanzen neben EGF beladen werden, die als Porogene fungieren und so erlauben, die Freigabe von EGF zu kontrollieren. Im Fall von nicht errodierbarem Matrixmaterial ist die Freigabe von EGF diffusionskontrolliert und kann durch die Art und die Menge der Porogene beeinflusst werden. Porogene Materialien sind z. B. wasserlösliche Polymere, wie beispielsweise Polyethylenglykol, verschiedene Polysaccharide oder Proteine wie Kollagen oder Gelatine. Andere wasserlösliche Substanzen mit niedrigem Molekulargewicht können den gleichen Zweck erfüllen.

Materialien, die für die Herstellung von errodierbaren Systemen verwendet werden, sind beispielsweise wasserlösliche Polymere, die Matrizen in fester oder halbfester Form herstellen, die sich in der flüssigen Umgebung des Auges auflösen oder die bei Körpertemperatur aufweichen oder schmelzen. Beispiele für derartige Polymere sind Alginat oder Zellulosederivate, wie beispielsweise Methylzellulose. Ein bevorzugtes Material, das für die Herstellung von zersetzbaren Inserts verwendet werden kann, ist Alginat, das aus α-L-Guluronsäure und β-D-Mannuronsäure besteht. Das Monomerverhältnis kann verwendet werden, um die Eigenschaften wie mechanische Stabilität und Arzneimittelfreigaberate zu kontrollieren. Aufgrund des Vorhandenseins von CarboxylSäuregruppen sind Alginate geladen und können durch Hinzufügung von Kationen vernetzt werden. Als Kationen können Kalzium oder Magnesium dienen. Der Grad der Vernetzung erlaubt, die Freigaberate von EGF sowie die Erosionsstabilität des Inserts zu kontrollieren. Ein besonderer Fall von errodierbaren Polymeren sind abbaubare Polymere, die einer Hydrolyse in einer wässrigen Umgebung unterzogen werden und in wasserlösliche Monomere und Oligomere zerfallen. Substanzen, die sich über thermische Veränderungen zersetzen, die aufweichen oder schmelzen, sind beispielsweise Fette wie Triglyzerine oder Phospholipide.

Um die Proteinfreigaberate aus den Filmen zu reduzieren oder die Freigaberaten derart zu kontrollieren, dass z. B. eine mehr lineare Freigabekinetik erzielt wird, können verschiedene Filme kombiniert werden. Derartige Laminate bestehen mindestens aus zwei Schichten, die sich voneinander entweder im Matrixmaterial, aus dem sie bestehen und/oder ihrer individuellen EGF-Beladung unterscheiden. Ein Beispiel für ein solches Laminat ist eine Konstruktion, die aus einer zentralen Schicht mit hohem EGF-Gehalt besteht, die auf beiden Seiten durch Filmschichten bedeckt ist, die einen geringen EGF-Gehalt besitzen. Eine sandwichartige Struktur mit abwechselnden Sequenzen von EGF-beladenen und EGF-freien Schichten ist ein weiteres Beispiel für ein solches Laminat.

Die Laminate können z. B. durch Bedrucken oder Aufsprühen verschiedener Matrixlösungen aufeinander hergestellt werden, durch Koazervation oder durch Tauchen der vorgefertigten Filme in entsprechende Lösungen oder die Schmelze von Materialien, die auf das Laminat aufgebracht werden sollen. Im letztgenannten Fall werden Polymerlösungen aus Polyelektrolyten hergestellt, die entgegengesetzt geladen sind. Die Ladungsinteraktionen führen zu einem Niederschlag der Polymerketten. Eingebundenes EGF wird daher immobilisiert in den entstehenden Polymermatrizen. Um abbaubare Systeme zu erzielen, muss wenigstens eines der Polyelektrolyte ein abbaubares Polymer sein oder eine dritte Komponente, die wasserlöslich oder biologisch abbaubar ist, wird der Mischung hinzugefügt. Beispiele für Polyelektrolytkombinationen sind Natriumalginat/Chitosan, Natriumalginat/Gelatine und Arabischer Gummi/Gelatine.

Eine Alternative zu Filmen und Laminaten sind
Reservoirsysteme, mittels derer die Abgabe von EGF von einem Insert kontrolliert werden kann. Reservoirsysteme bestehen z. B. aus einer flüssigen oder halbfesten EGF-Dispersion, die durch eine Membran umschlossen ist, die die EGF-Freigaberate kontrolliert. Ein weiteres Beispiel für ein EGF-Reservoir sind feste, wasserlösliche Mixturen, die beispielsweise durch Gefriertrocknung erzielt werden können und die sich nach dem Einsetzen des Inserts in ein flüssiges oder halbfestes System auflösen.

Die die Freigaberate kontrollierenden Membranen können wiederum zersetzbare Polymere wie z. B. Alginat sein, das mit Kalziumionen vernetzt ist, um die Stabilität zu erhöhen. Nicht zersetzbare mikroporöse Polymere können als Diffusionsbarrieren verwendet werden, um die Freigabe von EGF aus dem Reservoir zu steuern.

Filme, Laminate und Reservoirsysteme können darüber hinaus in Verbindung mit zusätzlichen Trägern verwendet werden, die die Freigabe von EGF kontrollieren. Partikel mit einer Größe von weniger als 100 µm, wie beispielsweise Mikrosphären, Nanopartikel oder Liposomen können mit EGF beladen sein, um die Freigaberate zu kontrollieren. Wenn derartige Partikel direkt ins Auge gegeben werden, gehen sie gewöhnlich recht rasch verloren. Durch ihr Einbetten in ein Insert, wie beispielsweise einen Film, ein Laminat oder eine reservoirartige Vorrichtung, ist es möglich, dieses Problem zu überwinden. Bei der Herstellung werden derartige Systeme einfach in der Matrixlösung dispergiert oder in der Schmelze des Materials, das für die Produktion des Inserts verwendet wird. Diese partikulären Systeme können genau so gut in Filme, Laminate oder Reservoirsysteme eingebunden werden.

Darüber hinaus gibt es eine Anzahl von weiteren zusätzlichen Freigabesysteme, die für die lokale Abgabe von EGF von einem Augeninsert verwendet werden können, die jedoch nicht in die Gruppe Film, Laminat oder Reservoirsystem einklassifiziert werden können.

In situ-gelierende Systeme sind vor der Anwendung im Auge flüssige Präparate und können daher leicht durch einen Patienten angewendet werden. Wenn sie in den Tränensack eingeführt sind, steigert sich ihre Viskosität oder sie verfestigen sich und bilden dabei ein EGF-reiches Depot. Beispiele für derartige Systeme sind Poloxamer-Lösungen, die unter Temperaturanstieg während der Anwendung gelieren. Ein weiteres Beispiel sind Polyelektrolyte, die ihre Ladung aufgrund von pH-Änderungen ändern, wie beispielsweise Polyacrylate und daher in einer wässrigen Umgebung ausfallen.

EGF kann darüber hinaus von Mikrochips freigegeben werden, die die Freigabe des Arzneistoffs von kleinen Reservoirs, die entsprechend einer vorprogrammierten Zeitstruktur geöffnet werden, durchführen. Beispielsweise kann die Freigabe über einen programmierbaren Read-Only Speicher (EPROM) auf dem gleichen Mikrochip durchgeführt werden (US-PS 5,797,898). Es ist darüber hinaus möglich, ein derartiges Freigabesystem mit einem Biosensor zu koppeln, der das lokalen EGF-Niveau misst und es so durch eine anpassbare Freigaberate konstant hält.

Schließlich können osmotische Pumpen eingesetzt werden.

Osmotische Pumpen besitzen beispielsweise ein EGF-Reservoir, das eine osmotisch aktive Substanz neben der EGF-Dosis enthält und das durch eine semi-permeable Membran umschlossen ist. Nachdem ein derartiges System ins Auge eingesetzt ist, tritt Wasser durch die semi-permeable Membran ein und beginnt, EGF und die osmotisch aktive Substanz aufzulösen oder zu verdünnen. Durch den Anstieg des osmotischen Drucks wird EGF aus dem Reservoir herausgedrückt. EGF verlässt dabei das System durch die Membran, die beispielsweise mikroporös ist oder kleine Öffnungen beinhaltet.

Im Folgenden soll die Erfindung anhand von Beispielen und einer Zeichnung näher erläutert werden.

### Beispiel: Herstellung eines EGF-haltigen Alginatfilms:

### 1. Herstellung eines Alginatgels:

1 g Natriumalginat (Protanal SF 120, Fa. Provona Biopolymer, Norwegen), wird exakt ausgewogen in ein 100 ml-Röhrchen mit Deckel gegeben. 99 g einer sterilen intraokkularen Waschlösung (BSS sterile intraokkular Lösung Pharmacia & Upjohn, Niederlande) wird hinzugegeben. Während des Quellens des Alginatpulvers wird die Mischung vorsichtig durch einen Magnetrührer bewegt. Nachdem sich das Alginat aufgelöst hat, werden 150 mg CaCl₂ x 2H₂O hinzugegeben und das Gel wird für wenigstens weitere 24 Stunden gerührt, bis es klar oder opaq erscheint und frei von Partikeln ist.

### 2. Herstellung einer EGF-Lösung:

1 mg EGF (rEGF, Biomol, Hamburg, Deutschland) wird in 1 ml doppelt destilliertem Wasser gelöst. Nach der vollständigen Auflösung werden weitere 9 ml Wasser hinzugefügt.

### 3. Herstellung eines EGF-geladenen Alginatfilms:

5,50 g Alginatgel werden in eine Petrischale abgewogen (7 cm Durchmesser, 4 cm Höhe). 1 ml EGF-Lösung (EGF-Gehalt: 100 µg/ml) werden in das Gel eingemischt unter Rühren mit einem Glasstab. Die resultierende Mischung wird in eine Teflon-Form (4 cm Durchmesser, 5 cm Höhe) gegeben. Der Film wird unter Umgebungsbedingungen in einer Arbeitsbank mit laminarem Luftfilm über 48 Stunden getrocknet.

### 4. Vernetzung des Films und Herstellung des Inserts:

Der getrocknete EGF-geladene Alginatfilm wird aus der Teflonform entfernt und für 60 Sekunden in eine 5 %-ige (w/w) wässrige Kalziumchloridlösung eingetaucht. Der Film wird wiederum in einer Arbeitsbank mit laminarem Luftfilm über 24 Stunden getrocknet. Ovale Inserts (r₁ = 9 mm lang, r₂ = 4 mm) werden ausgeschnitten oder ausgestanzt aus dem Film, der eine abschließende Dicke von ca. 100 µm aufweist.

Anhand einer Zeichnung sollen nun abschließend mögliche Formen der Inserts dargestellt werden.

Es zeigen:
- Figur 1: ein rundes Insert,
- Figur 2: ein ovales Insert,
- Figur 3: ein zweischichtiges Insert,
- Figur 4: ein dreischichtiges Insert,
- Figur 5: ein Insert mit EGF-geladenen und EGF-freien Schichten,
- Figur 6: eine reservoirartige Vorrichtung,
- Figur 7: ein weiteres Insert und
- Figur 8: ein Insert in Form einer osmotischen Pumpe.

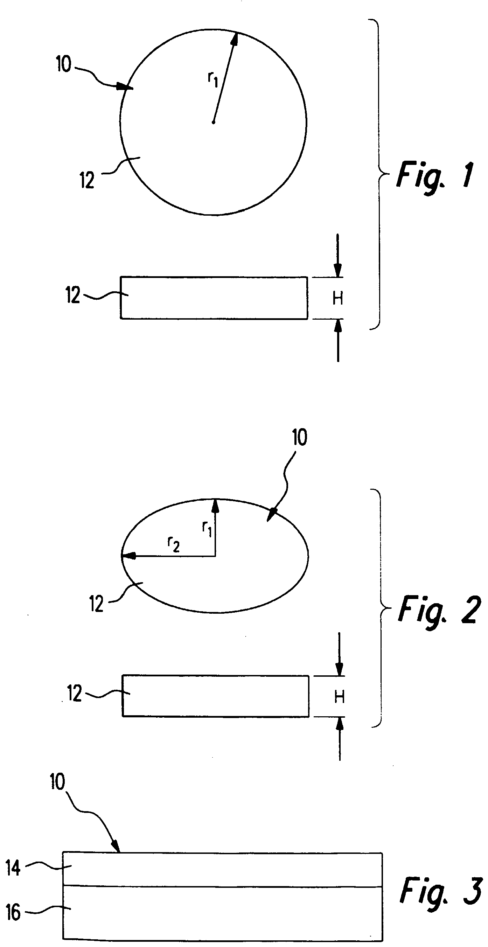

Figur 1 und 2 zeigen zwei verschiedene Formen, nämlich eine runde sowie eine ovale Form eines Inserts 10, das im übrigen zylindrisch gestaltet ist und eine Höhe H = 1 mm aufweist. Hierbei ist EGF in das Matrixmaterial 12 eingelegt. Das runde Insert 10 besitzt dabei einen Radius r₁ = 1 mm. Das ovale Insert weist einen Radius r₁ = 1,5 mm und einen Radius r₂ = 2 mm auf.

Figur 3 zeigt ein Insert 10, das beispielsweise die Form gemäß den Figuren 1 und 2 aufweisen kann, bestehend aus zwei verschiedenen Schichten 14, 16, wobei die obere Schicht 14 EGF-frei ist und die untere Schicht 16 mit EGF geladen ist.

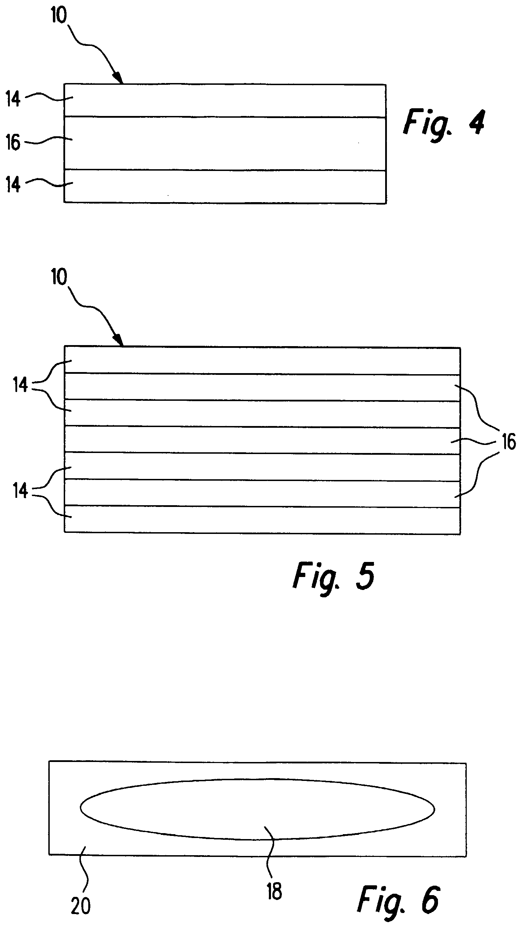

Figur 4 beschreibt einen ähnlichen Aufbau, wobei hier die EGF-geladene Schicht 16 durch zwei EGF-freie Schichten 14 eingeschlossen wird. Hierdurch kann die Freigabe von EGF an das Auge im Tränensack nach Einsetzen des Inserts 10 in denselben besser kontrolliert werden.

Einen erweiterten Aufbau zeigt beispielsweise Figur 5, in der sich EGF-freie Schichten 14 und EGF-geladene Schichten 16 abwechseln, wobei drei EGF-geladene Schichten 16 zwischen den EGF-freien Schichten 14 eingebettet sind.

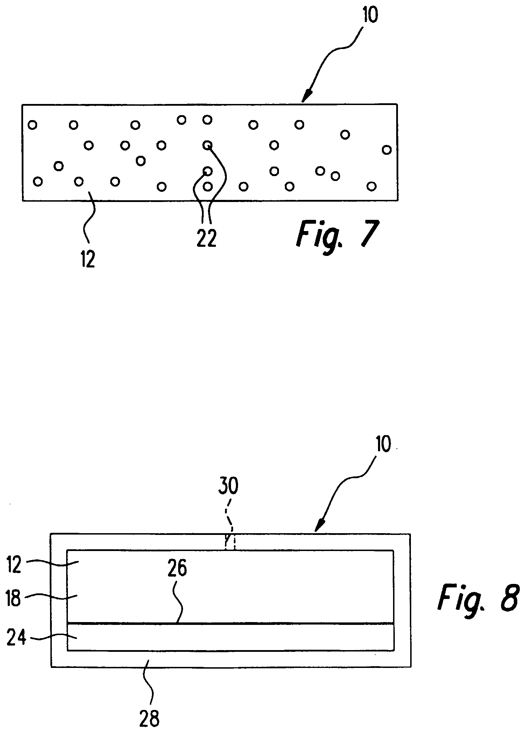

Figur 6 zeigt nun ein Reservoirsystem, wie es zuvor beschrieben wurde, wobei ein Reservoir 18 mit einer EGF-haltigen Lösung durch eine Membran 20 umschlossen ist, die die Abgabe von EGF an das Auge kontrolliert und steuert. Eine weitere Möglichkeit zur Einlagerung von EGF in eine Matrix 12 besteht darin, dass EGF-geladene Partikel 22 in einer Matrix 12 dispergiert sind und die Matrix 12 die Freigabe des EGF kontrolliert (Figur 7).

Schließlich zeigt die letzte Figur 8 eine weitere Ausgestaltung eines Inserts 10, bei der das Insert 10 als osmotische Pumpe gestaltet ist, wobei das Insert 10 eine EGF-geladene Matrix 12 umfasst sowie eine osmotisch aktive Substanz 24 und die EGF-geladene Matrix 12 von der osmotisch aktiven Substanz 24 durch eine impermeable Membran 26 getrennt ist. Die EGF-geladene Matrix 12 und die osmotisch aktive Substanz 24 sind gemeinsam durch eine semi-permeable Membran 28 umschlossen. Wird das Insert 10 nun in den Tränensack gegeben, so dringt Wasser durch die semi-permeable Membran 28 ein, und beginnt das EGF und die osmotisch aktive Substanz 24 zu verdünnen. Durch diesen gestiegenen osmotischen Druck wird EGF durch eine Öffnung 30 in der semi-permeablen Membran 28 aus dem Reservoir 18 hinausgedrückt und gelangt so in den Tränensack und damit ins Auge.

## Patentansprüche

1. Insert zur Behandlung von trockenen Augen, wobei das Insert in den Tränensack einlegbar oder auf die Hornhaut aufsetzbar ist und in dem Proteine und/oder Peptide in an das Auge freisetzbarer Form eingelagert sind, wobei die Proteine und/oder Peptide, Wachstumsfaktoren insbesondere Epidermal Growth Factor (EGF) und/oder Zytokine sind, **dadurch gekennzeichnet, dass** das Insert aus einer oder mehreren Schichten mit verschiedenen Protein- und/oder Peptid-Gehalten und/oder Matrixmaterialien aufgebaut ist, wobei insbesondere Protein- und/oder Peptid-beladene und Protein- und/oder Peptid-freie Schichten vorgesehen ist.

2. Insert zur Behandlung von trockenen Augen, wobei das Insert in den Tränensack einlegbar oder auf die Hornhaut aufsetzbar ist und in dem Proteine und/oder Peptide in an das Auge freisetzbarer Form eingelagert sind, wobei die Proteine und/oder Peptide, Wachstumsfaktoren insbesondere Epidermal Growth Factor (EGF) und/oder Zytokine sind, **dadurch gekennzeichnet, dass** das Insert ein Reservoir umfasst, das Proteine und/oder Peptide enthält und wobei das Reservoir durch eine Membran umschlossen ist und wobei insbesondere neben den Proteinen und/oder Peptiden freigebbare Kalziumionen vorhanden sind und wobei das Insert einen Mikrochip beinhaltet, der die Freigabe der Proteine und/oder Peptide aus Reservoirs steuert.

3. Insert zur Behandlung von trockenen Augen, wobei das Insert in den Tränensack einlegbar oder auf die Hornhaut aufsetzbar ist und in dem Proteine und/oder Peptide in an das Auge freisetzbarer Form eingelagert sind, wobei die Proteine und/oder Peptide, Wachstumsfaktoren insbesondere Epidermal Growth Factor (EGF) und/oder Zytokine sind, **dadurch gekennzeichnet, dass** das Insert ein in-situ Gelsystem umfasst, das Proteine und/oder Peptide enthält, welches vor der Verabreichung flüssig ist und am Anwendungsort durch den pH-Wert oder eine Temperaturänderung geliert, wobei das in-situ-Gelsystem im verfestigten Zustand insbesondere ein Protein- und/oder Peptid-haltiges Depot bildet und wobei insbesondere neben Proteinen und/oder Peptiden freigebbare Kalziumionen vorhanden sind.

4. Insert nach einem der Ansprüche 1 bis 3, wobei das Insert eine mit Proteinen und/oder Peptiden beladene Matrix umfasst, wobei die Proteine und/oder Peptide in der Matrix insbesondere gelöst oder suspendiert sind.

5. Insert nach einem der Ansprüche 1 bis 4, wobei die Proteine und/oder Peptide in der Matrix in stabiler Form vorliegen und durch sie in stabiler Form abgebbar sind.

6. Insert nach Anspruch 4 oder 5, wobei die Matrix freigebbare Kalziumionen umfasst.

7. Insert nach einem der Ansprüche 1 bis 6, wobei die Proteine und/oder Peptide und/oder die Kalziumionen in einer linearen Funktion freigebbar sind.

8. Insert nach einem der Ansprüche 1 bis 7, wobei die Proteine und/oder Peptide an Partikel gebunden vorliegt.

9. Insert nach einem der Ansprüche 1 bis 8, wobei die Freigabe der Proteine und/oder Peptide mittels einer osmotischen Pumpe erfolgt.

10. Insert nach einem der Ansprüche 1 bis 9, wobei das Insert eine runde oder ovale zylindrische Form mit einem Durchmesser r₁ = 0,1 bis 20 mm und r₂ = 0,1 bis 20 mm und einer Dicke D von 1 µm bis 5 mm aufweist.

11. Insert nach Anspruch 10, wobei r₁ = 0,5 bis 18 mm und r₂ = 0,5 bis 10 mm und D = 10 µm bis 1 mm beträgt.

12. Insert nach einem der Ansprüche 1 bis 11 , wobei das Insert erodierbar ist.

13. Herstellung eines Inserts nach einem der Ansprüche 1 bis 12, wobei ein wasserlösliches Gel hergestellt wird, insbesondere ein Alginatgel, das mit einer Protein- und/oder Peptid-Lösung, insbesondere eine Wachstumsfaktorlösung und insbesondere einer EGF-Lösung vermischt wird, und einen Protein- und/oder Peptid-geladenen Film bildet, der getrocknet wird, wonach aus dem Film die Inserts ausgestanzt oder ausgeschnitten werden.

## Claims

1. Insert for the treatment of dry eye, wherein the insert can be inserted into the lacrimal sac or can be placed onto the cornea and in which proteins and/or peptides are embedded in a manner which allows their release into the eye, wherein the protein and/or peptide are growth factors and preferably epidermal growth factor (EGF) and/or cytokines, **characterized by** the insert being made from one or a plurality of layers having different protein and/or peptide contents and/or matrix materials, wherein, in particular, protein and/or peptide-charged and protein and/or peptide-free layers are provided.

2. Insert for the treatment of dry eye, wherein the insert can be inserted into the lacrimal sac or can be placed onto the cornea and in which proteins and/or peptides are embedded in a manner which allows their release into the eye, wherein the protein and/or peptide are growth factors and preferably epidermal growth factor (EGF) and/or cytokines, **characterized by** the insert including a reservoir which contains proteins and/or peptides with the reservoir being enclosed in a membrane, and wherein, in particular in addition to proteins and/or peptides, releasable calcium ions are present and wherein the insert contains a microchip which controls dispensing of the protein and/or peptide out of the reservoir.

3. Insert for the treatment of dry eye, wherein the insert can be inserted into the lacrimal sac or can be placed onto the cornea and in which proteins and/or peptides are embedded in a manner which allows their release into the eye, wherein the protein and/or peptide are growth factors and preferably epidermal growth factor (EGF) and/or cytokines, **characterized by** the insert including an in-situ gel system which contains proteins and/or peptides which is liquid prior to use and which gels at the application location in response to pH values or a temperature change, wherein the in-situ gel system in the solid state, in particular, forms a protein and/or peptide containing deposit and wherein, in particular, in addition to proteins and/or peptides, dischargeable calcium ions are also present.

4. Insert according to claim 1 to 3, wherein the insert includes a matrix charged with proteins and/or peptides, wherein the proteins and/or peptides are preferably dissolved or suspended in the matrix.

5. Insert according to claims 1 to 4, wherein the proteins and/or peptides are present in the matrix in a stable form and can be released thereby in stable form.

6. Insert according to claims 4 or 5, wherein the matrix includes releasable calcium ions.

7. Insert according to any one of the claims 1 to 6, wherein the protein and/or peptides and/or the calcium ions can be dispensed in a linear function.

8. Insert according to any one of the claims 1 to 7, wherein the protein and/or peptide are bound to particles.

9. Insert according to any one of the claims 1 to 8, wherein the release of the proteins and/or peptides is effected by means of an osmotic pump.

10. Insert according to any one of the claims 1 to 9, wherein the insert has a round or oval cylindrical shape having a diameter r1 = 0.1 to 20 mm and r2 = 0.1 to 20 mm and a thickness D of between 1 µm to 5 mm.

11. Insert according to claim 10, wherein r1 = 0.5 to 18 mm, r2 = 0.5 to 10 mm and D = 10 µm to 1 mm.

12. Insert according to any one of the claims 1 to 11, wherein the insert is degradable.

13. Manufacture of an insert according to anyone of the claims 1 to 12, wherein a water soluble gel is produced, in particular an alginate gel, which is mixed with a protein and/or peptide solution, in particular a growth factor solution and, in particular, an EGF solution and a protein and/or peptide charged film is formed which is dried with inserts subsequently being cut or punched out of the film.

## Revendications

1. Insert destiné au traitement de la sécheresse oculaire, dans lequel l'insert peut être inséré dans le sac lacrymal ou placé sur la cornée, et dans lequel des protéines et/ou des peptides sont incorporé(e)s sous une forme libérable au niveau de l'oeil, dans lequel les protéines et/ou les peptides sont des facteurs de croissance, en particulier le facteur de croissance épidermique (EGF) et/ou des cytokines, **caractérisé en ce que** l'insert est composé d'une ou de plusieurs couches ayant des teneurs différentes en protéines et/ou en peptides et/ou de matériaux matriciels, dans lequel en particulier des couches chargées de protéines et/ou de peptides et/ou des couches exemptes de protéines et/ou peptides sont fournies.

2. Insert destiné au traitement de la sécheresse oculaire, dans lequel l'insert peut être inséré dans le sac lacrymal ou placé sur la cornée, et dans lequel des protéines et/ou des peptides sont incorporé(e)s sous une forme libérable au niveau de l'oeil, dans lequel les protéines et/ou les peptides sont des facteurs de croissance, en particulier le facteur de croissance épidermique (EGF) et/ou des cytokines, **caractérisé en ce que** l'insert comprend un réservoir qui contient des protéines et/ou des peptides, et dans lequel le réservoir est entouré d'une membrane, et dans lequel en particulier, outre les protéines et/ou peptides, des ions calcium pouvant être libérés sont inclus, et dans lequel l'insert comprend une micropuce qui commande la libération des protéines et/ou des peptides à partir de réservoirs.

3. Insert destiné au traitement de la sécheresse oculaire, dans lequel l'insert peut être inséré dans le sac lacrymal ou placé sur la cornée, et dans lequel des protéines et/ou des peptides sont incorporé(e)s sous une forme libérable au niveau de l'oeil, dans lequel les protéines et/ou les peptides sont des facteurs de croissance, en particulier le facteur de croissance épidermique (EGF) et/ou des cytokines, **caractérisé en ce que** l'insert comprend un système de gel in situ qui contient des protéines et/ou des peptides, qui est liquide avant administration et gélifié au niveau du lieu d'utilisation par le pH ou un changement de température, dans lequel le système de gel in situ, à l'état solidifié, forme en particulier un dépôt contenant des protéines et/ou des peptides et dans lequel en particulier, en plus des protéines et/ou des peptides, des ions calcium libérables sont inclus.

4. Insert selon l'une des revendications 1 à 3, dans lequel l'insert comprend une matrice chargée de protéines et/ou de peptides, dans lequel en particulier les protéines et/ou les peptides de la matrice sont dissoutes/dissous ou en suspension.

5. Insert selon l'une des revendications 1 à 4, dans lequel les protéines et/ou peptides sont présent(e)s dans la matrice sous une forme stable et peuvent être délivrés à travers celle-ci sous une forme stable.

6. Insert selon la revendication 4 ou 5, dans lequel la matrice comprend des ions calcium libérables.

7. Insert selon l'une des revendications 1 à 6, dans lequel les protéines et/ou les peptides et/ou les ions calcium peuvent être libérés dans une fonction linéaire.

8. Insert selon l'une quelconque des revendications 1 à 7, dans lequel les protéines et/ou les peptides sont liés à des particules.

9. Insert selon l'une des revendications 1 à 8, dans lequel la libération des protéines et/ou des peptides survient au moyen d'une pompe osmotique.

10. Insert selon l'une des revendications 1 à 9, dans lequel l'insert a une forme cylindrique, ronde ou ovale avec un diamètre r₁ = 0,1 à 20 mm et r₂ = 0,1 à 20 mm et une épaisseur D de 1 µm à 5 mm.

11. Insert selon la revendication 10, dans lequel r₁ = 0,5 à 18 mm et r₂ = 0,5 à 10 mm et D = 10 µm à 1 mm.

12. Insert selon l'une quelconque des revendications 1 à 11, dans lequel l'insert est érodable.

13. Fabrication d'un insert selon l'une des revendications 1 à 12, dans lequel un gel hydrosoluble est préparé, en particulier un gel d'alginate qui est mélangé à une solution de protéines et/ou de peptides, en particulier une solution de facteur de croissance, et en particulier une solution d'EGF, et forme un film chargé de protéines et/ou de peptides qui va être séché, après quoi les inserts sont matricés ou découpés dans le film.
